# EUROPEAN PATENT SPECIFICATION

(11) **EP 3 793 737 B1**
(45) Date of publication and mention of the grant of the patent: **14.01.2026**
(21) Application number: 19804400.0
(22) Date of filing: 10.05.2019
(51) Int. Cl.: B01L 3/00, A61K 35/17, C12M 1/00, C12M 3/06

(54) **COMBINED PURIFICATION AND CONCENTRATION BY DETERMINISTIC LATERAL DISPLACEMENT WITH RECIRCULATION PRODUCT**
KOMBINIERTE REINIGUNG UND KONZENTRIERUNG DURCH DETERMINISTISCHE SEITLICHE VERDRÄNGUNG MIT EINEM REZIRKULATIONSPRODUKT
PURIFICATION ET CONCENTRATION COMBINÉES PAR DÉPLACEMENT LATÉRAL DÉTERMINISTE AVEC PRODUIT DE RECIRCULATION

(30) Priority: 13.05.2018 US 201862670839 P
(43) Date of publication of application: 24.03.2021
(73) Proprietor: Zeon Corporation, Tokyo 100-8246 (JP)
(72) Inventor: SKELLEY, Alison, Riverside, CA 92508 (US); WARD, Anthony, Santa Fe, CA 90267 (US); GANDHI, Khushroo, Palo Alto, CA 94306 (US); CAMPOS-GONZALEZ, Roberto, Carlsbad, CA 92009 (US)
(74) Representative: Maiwald GmbH
(86) International application number: PCT/US2019/031738
(87) International publication number: WO 2019/222049

(56) References cited:
- WO-A1-2017/035262
- WO-A1-2018/080997
- WO-A1-2018/080997
- WO-A1-2019/046052
- WO-A2-2010/129441
- US-A1- 2009 283 456
- US-A1- 2010 006 479
- US-A1- 2016 047 735

## Description

### Cross Reference to Related Applications

The present application claims the benefit of US. Provisional Patent Application No. 62/670,839, filed on May 13, 2018.

### Field of the Invention

The present invention is directed to methods of simultaneously purifying and concentrating cells or other particles by performing deterministic lateral displacement on microfluidic devices while recirculating product.

### Background of the Invention

Deterministic lateral displacement (DLD) typically uses two co-flowing liquids, one containing particles (the sample) and one that is a wash/collection fluid (the "running" or "wash" fluid). During DLD, particles above the critical diameter of the array are deflected into the wash fluid whereas particles below the critical diameter follow the flow direction of the sample stream. The concentration of particles in the final product (collected) stream is dependent on the input concentration of particles in the sample stream, the ratio of sample to wash fluid, and the percentage of the total volume that is collected as product. Obtaining high concentration factors using most current DLD procedures is possible but can increase the risk of contamination (particles lower than the critical diameter being collected in the product stream) or can require very long DLD arrays with extra redundancy to ensure efficient bumping into a narrow collection width.

It is also difficult to adjust the concentration of the product produced by DLD to achieve consistency on a run-by-run basis or to standardize the results obtained across multiple donor samples with different input cell counts. Samples can always be diluted, but concentration is far more challenging. Samples with different input concentrations cannot be standardized to a single output concentration without doing an up-front dilution that would be different for each sample.

Another problem with controlling product concentrations using current procedures is that relatively large volumes of wash fluid are usually required. Limiting these volumes should result in higher product concentrations, but there must always be enough wash fluid to process the entire sample volume.

WO2018080997 A1 discloses a method of separating target particles of a predetermined size from a sample by use of deterministic lateral displacement, wherein a population of separated particles can be continuously looped back into the same or a different device for further separation.

### Summary of the Invention

### General Description

The present invention is based on the concept that it is possible to control the final concentration of DLD products by recirculating product streams back onto microfluidic devices and applying them in the place of wash fluid. Initially both sample and wash fluid (also referred to herein more specifically as a wash buffer or reagent) are fed onto a device. Then, at a chosen point during the run, the inlet carrying wash fluid is sealed off, and product from an outlet of the device is fed through the inlet in the wash fluid's place. This can be accomplished using standard valves, with the recycled product stream being propelled using, for example, pressurized containers, peristaltic pumps, or syringe pumps.

During the time that collected product (P1) is being recycled onto the device, there are two processes occurring. First, sample flowing adjacent to the recycled product is being fractionated based on size such that large particles are deflected into the "wash stream" (now P1) and directed to the product outlet. Second, the number of particles in P1 is being increased thereby increasing product concentration. One way that this process may be characterized is in terms of a "concentration ratio," which is a ratio of the percentage of wash fluid in vs. percentage of product collected. A typical set of ratios might be: 60% sample and 40% wash fluid at the inlets, and 20% product and 80% waste at the outlet. Under these circumstances, if P1 is recycled as wash fluid, the particles in the wash stream will be concentrated by 2x (40%/20%) because they are still being deflected in the array and into the product collection stream. Therefore, the collected product (P2) contains both the P1 cells concentrated at 2x, in addition to the cells from the sample processed during that time period.

One advantage of this method is that the final concentration of the output product is dependent on the number of recirculations and the initial wash volume used. It is therefore easily adjustable. For example, with a donor sample that is very dilute, the operator may choose to run only a small volume of the sample against wash fluid, and then recirculate the product several times in order to process the remaining sample. As a result, a higher concentration factor may be obtained. This allows the operator (or instrumentation) to deliver any output concentration regardless of input concentration. It also means that instrumentation can be optimized to deliver a fixed product concentration needed for downstream assay steps independent of the input donor concentration. The user would simply input the initial donor concentration, and select a desired output concentration. Using this information, the instruments would adjust the number of recirculations to achieve the selected value.

Another advantage of this method is that concentration is achieved without requiring a separate concentrator device (either in-line, or run as a second pass). The concentration occurs on the same DLD used for the separation of the cells and the time needed to process the donor sample remains the same as it would be if the sample were run solely against wash buffer.

The volume of wash fluid required is also significantly less. Instead of requiring enough wash fluid to process the entire sample, the wash volume will be reduced by an amount corresponding to the volume of recycled product. For example, if recycling was initiated after about 50% of sample had been processed, the volume of wash fluid would be reduced by about half.

### Embodiments

In its first aspect, the invention is directed to a method of separating target cells or target particles of a predetermined size from a sample containing cells or particles of less than the predetermined size. The method comprises applying the sample and a wash fluid to a microfluidic device at separate inlets. The wash fluid may be water or an aqueous buffer, and may optionally comprise reagents that chemically react with sample components or antibodies, carriers or activators that interact specifically with target cells or target particles. As initially applied to devices, *i.e.,* before any recirculation of product, wash fluid should devoid of target cells or particles and without any of the cells or particles of less than the predetermined size.

The microfluidic device should be configured for deterministic lateral displacement (DLD), a process that involves flowing a sample through a specifically designed array of microposts that are tilted at a small angle from the direction of fluid flow (see, Davis, et al., Proc. Natl. Acad. Sci. USA 103:14779-14784 (2006); Inglis, et al., Lab Chip 6:655-658 (2006); Chen, et al., Biomicrofluidics. 9(5):054105 (2015)). Thus, the microfluidic device will have an array of obstacles arranged in rows, with each subsequent row of obstacles shifted laterally with respect to a previous row. The obstacles should be positioned so as to deflect target cells or particles to a first outlet (where they may be recovered as a target cell or particle product), and to direct cells or particles of less than the predetermined size to a second outlet (where they may be collected or discarded as waste).

DLD is performed by flowing the sample and wash fluid through the microfluidic device. During this process, at least a portion of the target cell or target particle product is recirculated from the first outlet to replace all, or at least a portion, of the wash fluid being applied to an inlet of the device. During, or at the end of, the procedure, a final product comprising target cells or particles is obtained from the first outlet.

To facilitate recirculation, the first outlet on the microfluidic device comprises, or is connected to, a valve that can be used to divert the target cell or target particle product to a conduit that recycles the product to an inlet on the microfluidic device. Similarly, the microfluidic device may have an inlet that comprises, or is connected to, a valve that can be used to switch the feed entering the device through the inlet from a conduit feeding wash fluid to a conduit feeding target cell or target particle product.

In some embodiments target cells or target particles being recirculated to a microfluidic plate are reacted with, or bound to, a carrier, antibody, fluorescent tag, activator or compound prior to, during or after being reapplied to the microfluidic device. In a preferred embodiment, target cells, e.g., leukocytes or, more specifically, T cells, are the target cells and are bound with specificity by carriers, antibodies or activators. As used in this context, the word "specificity" means that at least 100 (and preferably at least 1000) target cells will be bound by carrier relative to each non-target cell bound in a sample. Such binding may be used to promote cell division (in the case of activators), or to facilitate the assay or further purification of cells (in the case of antibodies).

Carriers may be used to alter the behavior of cells during **DLD** procedures. For example, cells exiting devices at one position may be bound to a carrier to form a complex that exits the same device at a different position. Thus, size based separations may be achieved that would otherwise not be possible. In this case, binding of carrier should be done "in a way that promotes DLD separation." This term, as used in the present context, means that the method must ultimately result in binding that exhibits specificity for a particular target cell type, that provides for an increase in size of the complex relative to the unbound cell of at least 2 µm (and alternatively at least 20, 50, 100, 200, 500 or 1000% when expressed as a percentage) and, in cases where therapeutic or other uses require free target cells, that allows the target cell to be released from complexes by chemical or enzymatic cleavage, chemical dissolution, digestion, due to competition with other binders, by physical shearing, e.g., using a pipette to create shear stress, or by other means. Carriers may also be bound in a way that complements DLD separation.

In order to determine concentration, cell or particle counts may be made of the target cell or target particle products. Based on this, a determination can be made as to whether to continue recycling product. Depending on the objectives of the party carrying out the process, relative to the concentration in the sample, cells or particles may be concentrated by a factor of at least 3 or by a factor of at least 10. In the case of cells, recirculation in combination with microfluidic processing will preferably be the sole method used for concentration during this process. This is especially true of cells that will be genetically engineered and/or used therapeutically.

The methods described herein can be used in the separation and concentration of cells derived from bioreactors, flasks, culture plates, culture bags, biological fluids or extracts, and preparations derived from tissues. Particularly preferred are leukocytes (preferably T cells, and most preferably CAR-T cells) or stem cells of a predetermined size. Samples containing these cells may be blood, compositions obtained by performing apheresis, leukapheresis, or leukoreduction, or leukopak preparations and will typically include platelets or red blood cells which are smaller than the predetermined size. Also, cells may be genetically engineered by any method used in the art, including transfection electrically, chemically or by means of nanoparticles or transduction using a virus.

In some instances, leukocytes or stem cells being recirculated may be bound to a carrier, antibody, or activator in a way that promotes or complements DLD separation. Binding should take place after cells have passed through the microfluidic device at least once and prior to, during or after being recirculated to the microfluidic device. In cases where cells are to be used therapeutically, the process of preparing the cells should not include a centrifugation step and recirculation should be performed until a concentration of cells is obtained that is sufficient to allow them to be therapeutically administered to a patient or for subsequent for processing. Similarly, addition of viral or genetic material in the concentration loop prior to release may be possible. In addition, it is preferred that in cases where a sample is obtained from a patient for the isolation of cells to be used therapeutically, no more than four hours elapse from the time that the obtaining of the sample is complete until processing of cells by DLD is complete.

It will be readily apparent to those of skill in the art that, with some modifications, the procedures described herein could be adapted to separating target cells or particles from contaminants of a larger size.

### Integrated Method for Making Purified Genetically Engineered Target Cells

In a preferred embodiment, the procedures described above can be used as part of a method for making genetically engineered target cells according to the appended claims 7 and 8. It is also preferred that no other means for concentrating target cells be used during this process other than the microfluidic separation with product recirculation procedure discussed above.

The term "cells of a predetermined size" as used herein refers to the size of the target cells chosen for separation. The size may either be experimentally determined or known from the art. For example, the size of human T cells is known in the art as are the sizes of platelets and red blood cells. Thus, a separation could be carried out in which T cells were the "target cells of a predetermined size" and the platelets and red blood cells were contaminant cells being separated. The term "predetermined size" may also be used in the context of the design of microfluidic devices for carrying out separations. For example the tem "critical size" or "predetermined size" of particles passing through an obstacle array may be used to describe the size limit of particles that are able to follow the laminar flow of fluid. Larger particles can, for example, be 'bumped' from the flow path of the fluid while particles having sizes lower than the critical size (or predetermined size) will not necessarily be displaced.

Many types of cells may be used for target cells, including therapeutically active cells such as lymphocytes and stem cells. T lymphocytes are especially preferred as these may be genetically engineered to produce the chimeric antigen receptors (CARs) on their surface and form therapeutically valuable chimeric antigen receptor T cells.

DLD may also be used in step c) to separate genetically engineered target cells from reagents, virus or other materials used in transforming or transfecting the target cells. In cases where the genetically engineered cells will be expanded in cell culture, (typically done in the production of CAR T cells) the materials used in transforming or transfecting the target cells may be removed in step e) while concurrently transferring the cells into growth medium. The cells, once transferred, can be collected and cultured or, alternatively, they can flow directly to the site where culturing will occur.

The wash fluid used in the methods discussed above may be water or an aqueous buffer and may comprise reagents that chemically react with cells, particles or other components in the wash fluid or comprise antibodies, carriers or activators that interact specifically with target cells.

In general, the directing of target cells exiting the device will be due to an outlet that either comprises a valve or that is connected to a valve. Depending on whether it is opened or closed, the valve will direct target cells: a) from the outlet to a conduit that recycles the cells to an inlet of the microfluidic device; or b) from the outlet to a site where the target cells are transformed or transfected to form genetically engineered target cells. Preferably, the valve is configured to electronically respond to a concentration of target cells below a desired concentration, PC, by directing the target cells to the conduit that recycles the cells to an inlet on the microfluidic device and to electronically respond to a concentration of target cells at or above PC by directing the target cells to a site where they are collected or to a site where they are transformed or transfected to form genetically engineered target cells.

The target cells being recirculated to the microfluidic device inlet may be reacted with, or bound to, a carrier, antibody, fluorescent tag, activator or compound prior to, during or after being reapplied to the microfluidic device. For example, leukocytes or stem cells may be bound to a carrier, antibody, or activator in a way that promotes or complements DLD separation.

Recirculation of target cells may be continued until, relative to the concentration in the sample, target cells are concentrated by a factor of at least 3, preferably by a factor of at least 5, and still more preferably by a factor of at least 10. It is also preferable that during the entire time from the obtaining of sample in step a) until the collection of cells, target cells are not centrifuged or frozen. In addition, sample will be preferably obtained from a patient that will eventually be treated by the cells produced and no more than 10 hours (preferably, no more than five hours) will elapse from the time that the obtaining of the sample is completed until step f) is completed.

The most preferred samples are blood or a composition that has been obtained by processing blood, e.g., by apheresis or leukapheresis. Samples of this type might be used, for example, to separate leukocytes from platelets and/or red blood cells.

### Brief Description of the Drawings

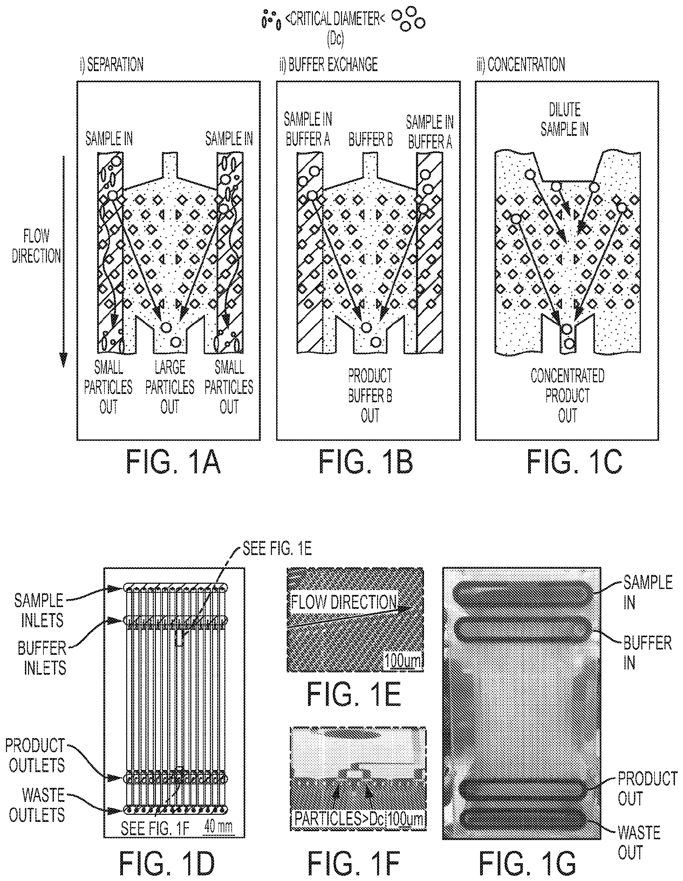

**Figures 1A-1G:** Figures 1A-1C illustrate different operating modes of one type of a DLD device. This includes: i) Separation (Fig. 1A), ii) Buffer Exchange (Fig. 1B) and iii) Concentration (Fig. 1C). In each mode, essentially all particles above a critical diameter are deflected in the direction of the array from the point of entry, resulting in size selection, buffer exchange or concentration as a function of the geometry of the device. In all cases, particles below the critical diameter pass directly through the device under laminar flow conditions and subsequently off the device at an outlet. DLD devices have been described in the literature along with methods of making and using the devices, see *e.g.,* US 2016/0139012; US 2017/0333900; US 2016/0047735; US 2017/0209864; US 2017/0248508; and US 2019/0071639.
   Figure 1D shows a 14 lane DLD design used in separation mode. The full length of the depicted array and microchannel is 75 mm and the width is 40 mm, each individual lane is 1.8 mm across. Figures 1E-1F are enlarged views of the plastic diamond post array and consolidating collection ports for the exits. Figure 1G depicts a photo of a leukapheresis product being processed using a device.

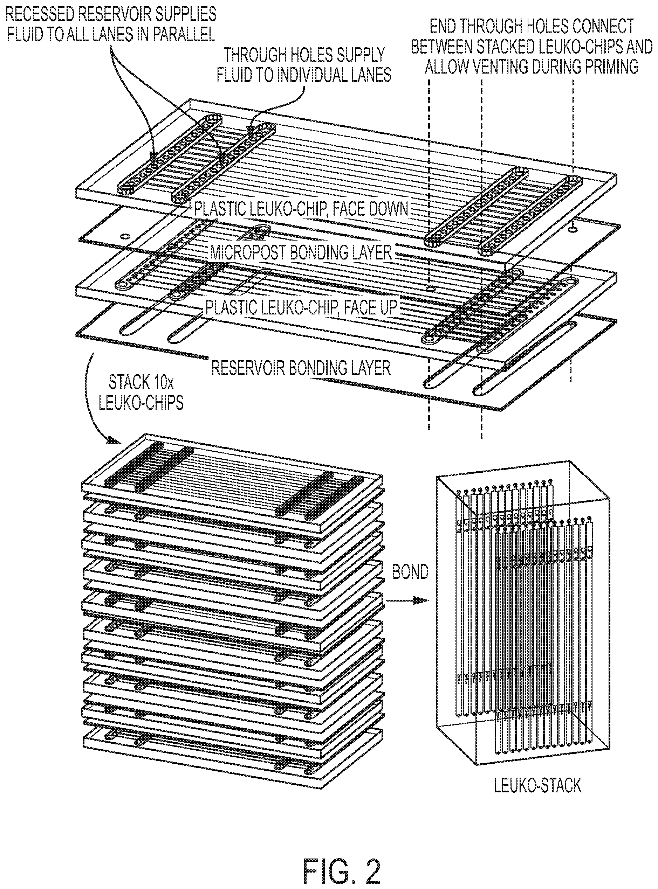

**Figure 2:** Figure 2 is a schematic showing how current individual chips have been designed to be stackable in layers to achieve throughput as demanded by any particular application.

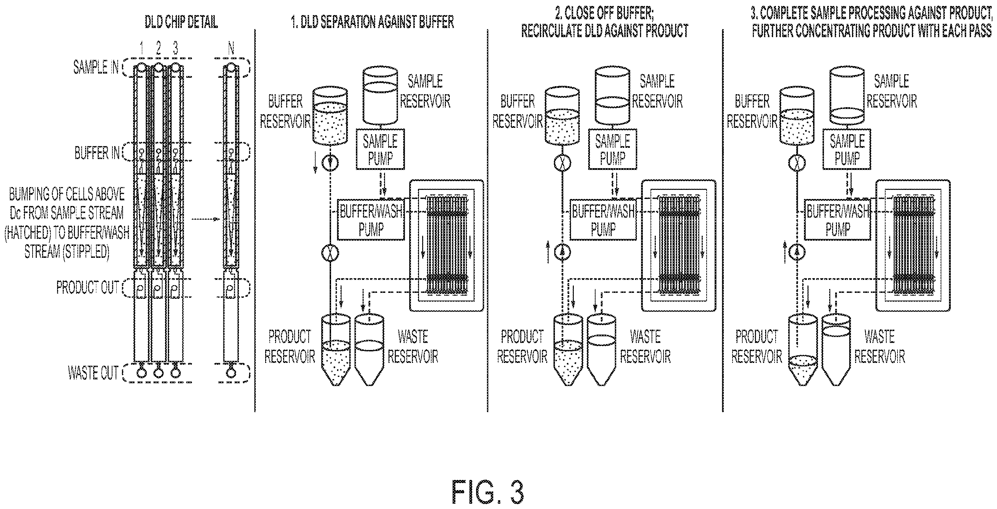

**Figure 3****:** The diagram on the far left of figure 3 illustrates the movement of cells during DLD. Buffer and sample are applied to the device at separate inlet ports. As the sample progresses toward the outlet, cells with a size greater than the critical size of the array move from the sample stream (outer, hatched portion of the channels) to the buffer stream (center, stippled portion of the channels) and eventually exit at the product outlet. Panels 1-3 show various steps in a DLD procedure in which there is a recycling of product. In Panel 1, sample (white in upper sample reservoir) and buffer (stippled in upper buffer reservoir) are applied to a microfluidic device through separate inlets. Product containing cells greater than the critical size of the array is collected from the product outlet (stippled in the bottom product reservoir) and waste is collected from the waste outlet (clear in the bottom waste reservoir). Note that the valve from the product reservoir to the buffer inlet is closed whereas the valve from the buffer reservoir to the buffer inlet is open. In Panel 2, the valve from the product reservoir to the buffer inlet has been opened and the valve from the buffer reservoir to the buffer inlet has been closed. As a result product is recycled back on to the microfluidic device. In Panel 3, processing has proceeded to near completion. Total volume of waste has increased and total volume of product has decreased.

### Illustrative Tables

Calculated results expected from a procedure are shown in the tables below. The numbers are based on a selected set of input and output conditions for a 14 lane microfluidic device. It should be recognized that microfluidic devices with a different layout (and ratios of inlets/outlets) will have numbers that scale differently.

**Table 1: Assumptions Table 2: Process sample entirely by DLD**

| | |
|---|---|
| DLD throughput, mL/hr | 1000 |
| Donor volume, mL | 400 |
| Input WBC x10^6 cells/mL | 20 |
| Dilution factor | 0.2 |
| Input % sample | 60 |
| Input % buffer | 40 |
| Output % product | 20 |
| Output % waste | 80 |

| | |
|---|---|
| Input volume, mL | 2000 |
| Sample throughput, mL/hr | 600 |
| Time to process, hr | 3.3 |
| Buffer volume needed, mL | 1333.3 |
| Final product volume, mL | 666.7 |
| Final concentration x 10^6/mL | 12 |
| Concentration factor from donor | 0.6 |

**Table 3: Process by DLD, then recirculate second pass in concentration mode**

| | |
|---|---|
| Second stage input volume, mL | 666.7 |
| Second stage concentration | 12 |
| Time to process second stage, hr | 0.7 |
| Final product volume, mL | 133.3 |
| Final concentration x 10^6/mL | 60 |
| Elapsed time, | 4 |
| Buffer volume needed, mL | 1333.3 |
| Concentration factor from donor | 3 |

**Table 4: Process by DLD; initially use buffer but then recirculate DLD product as buffer for remainder**

| | | | | | | |
|---|---|---|---|---|---|---|
| Input vol. mL | 2000 | | | | | |
| Sample throughput, mL/hr | 600 | | | | | |
| Time to process, hr | 3.3 | | | | | |
| **Fraction processed against buffer** | **0.8** | **0.75** | **0.7** | **0.65** | **0.6** | **0.55** |
| Volume sample processed, mL | 1600 | 1500 | 1400 | 1300 | 1200 | 1100 |
| Volume buffer needed, mL | 1066.7 | 1000.0 | 933.3 | 866.7 | 800.0 | 733.3 |
| Volume P1 after initial pass, mL | 533.3 | 500.0 | 466.7 | 433.3 | 400.0 | 366.7 |
| Remaining sample to be processed | 400.0 | 500.0 | 600.0 | 700.0 | 800.0 | 900.0 |
| Total Volume "buffer" needed, mL | 266.7 | 333.3 | 400.0 | 466.7 | 533.3 | 600.0 |
| Number of recirculations required | 0.50 | 0.7 | 0.8 | 1.1 | 1.5 | 1.8 |
| Volume after 1st recirculation | | | | 216.7 | 200.0 | 183.3 |
| Volume "buffer" still needed | | | | 33.3 | 133.3 | 233.3 |
| Volume after processing 2nd pass | | | | 16.7 | 66.7 | 116.7 |
| Volume "buffer" still needed | | | | | | 50.0 |
| Volume after 2nd recirculation | | | | | | 25.0 |
| Final product volume, mL | 400.0 | 333.3 | 272.2 | 200.0 | 133.3 | 91.7 |
| Final concentration x10^6/mL | 50.0 | 60.0 | 73.5 | 100.0 | 150.0 | 218.2 |
| Concentration factor from donor | 2.5 | 3.0 | 3.7 | 5.0 | 7.5 | 10.9 |

### Summary

| **Process** | **Donor Vol.** | **Final Vol.** | **WBC** | **Time (hr)** | **Final WBC** | **Conc. Factor** |
|---|---|---|---|---|---|---|
| DLD only | 400 | 20 | 666.7 | 12.0 | 3.3 | 0.6 |
| DLD then concentrate (2nd pass) | 400 | 20 | 133.3 | 60.0 | 4.0 | 3.0 |
| Process 0.8% by DLD, then recirculate product | 400 | 20 | 400.0 | 50.0 | 3.3 | 2.5 |
| Process 0.65% by DLD, then recirculate product | 400 | 20 | 200 | 100 | 3.3 | 5.0 |
| Process 0.55% by DLD, then recirculate product | 400 | 20 | 91.7 | 218.2 | 3.3 | 10.9 |

### Definitions

Apheresis: As used herein, this term refers to a procedure in which blood from a patient or donor is separated into its components, e.g., plasma, white blood cells and red blood cells. More specific terms are "plateletpheresis" (referring to the separation of platelets) and "leukapheresis" (referring to the separation of leukocytes). In this context, the term "separation" refers to the obtaining of a product that is enriched in a particular component compared to whole blood and does not mean that absolute purity has been attained.

CAR T cells: The term "CAR" is an acronym for "chimeric antigen receptor." A "CAR T cell" is therefore a T cell that has been genetically engineered to express a chimeric receptor. Methods for making and using CAR T cells are well known in the art. Procedures have been described in, for example, US 9,629,877; US 9,328,156; US 8,906,682; US 2017/0224789; US 2017/0166866; US 2017/0137515; US 2016/0361360; US 2016/0081314;US 2015/0299317; and US 2015/0024482.

CAR T cell therapy: This term refers to any procedure in which a disease is treated with CAR T cells. Diseases that may be treated include hematological and solid tumor cancers, autoimmune diseases and infectious diseases.

Carrier: As used herein, the term "carrier" refers an agent, *e.g.,* a bead, or particle, made of either biological or synthetic material that is added to a preparation for the purpose of binding directly or indirectly *(i.e.,* through one or more intermediate cells, particles or compounds) to some or all of the compounds or cells present. Carriers may be made from a variety of different materials, including DEAE-dextran, glass, polystyrene plastic, acrylamide, collagen, and alginate and **will** typically have a size of 1-1000 µm. They may be coated or uncoated and have surfaces that are modified to include affinity agents (e.g., antibodies, activators, haptens, aptamers, particles or other compounds) that recognize antigens or other molecules on the surface of cells. The carriers may also be magnetized and this may provide an additional means of purification to complement DLD and they may comprise particles (e.g., Janus or Strawberry-like particles) that confer upon cells or cell complexes non-size related secondary properties. For example, the particles may result in chemical, electrochemical, or magnetic properties that can be used in downstream processes, such as magnetic separation, electroporation, gene transfer, and/or specific analytical chemistry processes. Particles may also cause metabolic changes in cells, activate cells or otherwise promote cell division.

Carriers that bind "in a way that promotes DLD separation": This term, refers to carriers and methods of binding carriers that affect the way that, depending on context, a cell, protein or particle behaves during DLD. Specifically, "binding in a way that promotes DLD separation" means that: a) the binding must exhibit specificity for a particular target cell type, protein or particle; and b) must result in a complex that provides for an increase in size of the complex relative to the unbound cell, protein or particle. In the case of binding to a target cell, there must be an increase of at least 2 µm (and alternatively at least 20, 50, 100, 200, 500 or 1000% when expressed as a percentage). In cases where therapeutic or other uses require that target cells, proteins or other particles be released from complexes to fulfill their intended use, then the term "in a way that promotes DLD separation" also requires that the complexes permit such release, for example by chemical or enzymatic cleavage, chemical dissolution, digestion, due to competition with other binders, or by physical shearing (e.g., using a pipette to create shear stress) and the freed target cells, proteins or other particles must maintain activity; e.g., therapeutic cells after release from a complex must still maintain the biological activities that make them therapeutically useful.

Carriers may bind "in a way that complements DLD separation": This term refers to carriers and methods of binding carriers that change the chemical, electrochemical, or magnetic properties of cells or cell complexes or that change one or more biological activities of cells, regardless of whether they increase size sufficiently to promote DLD separation. Carriers that complement DLD separation also do not necessarily bind with specificity to target cells, *i.e.,* they may have to be combined with some other agent that makes them specific or they may simply be added to a cell preparation and be allowed to bind non-specifically. The terms "in a way that complements DLD separation" and "in a way that promotes DLD separation" are not exclusive of one another. Binding may both complement DLD separation and also promote DLD separation. For example a polysaccharide carrier may have an activator on its surface that increases the rate of cell growth and the binding of one or more of these carriers may also promote DLD separation. Alternatively binding may just promote DLD separation or just complement DLD separation.

Target cells: As used herein "target cells" are the cells that various procedures described herein require or are designed to purify, collect, engineer etc. What the specific cells are will depend on the context in which the term is used. For example, if the objective of a procedure is to isolate a particular kind of stem cell, that cell would be the target cell of the procedure.

Isolate, purify: Unless otherwise indicated, these terms, as used herein, are synonymous and refer to the enrichment of a desired product relative to unwanted material. The terms do not necessarily mean that the product is completely isolated or completely pure. For example, if a starting sample had a target cell that constituted 2% of the cells in a sample, and a procedure was performed that resulted in a composition in which the target cell was 60% of the cells present, the procedure would have succeeded in isolating or purifying the target cell.

Deterministic Lateral Displacement: As used herein, the term "Deterministic Lateral Displacement" or "DLD" refers to a process in which particles are deflected on a path through an array, deterministically, based on their size in relation to some of the array parameters. Processes are generally described herein in terms of continuous flow (DC conditions; *i.e.,* bulk fluid flow in only a single direction). However, DLD can also work under oscillatory flow (AC conditions; *i.e.,* bulk fluid flow alternating between two directions).

Critical size: The "critical size" or "predetermined size" of particles passing through an obstacle array describes the size limit of particles that are able to follow the laminar flow of fluid. Particles larger than the critical size can be 'bumped' from the flow path of the fluid while particles having sizes lower than the critical size (or predetermined size) will not necessarily be so displaced. When a profile of fluid flow through a gap is symmetrical about the plane that bisects the gap in the direction of bulk fluid flow, the critical size can be identical for both sides of the gap; however when the profile is asymmetrical, the critical sizes of the two sides of the gap can differ.

Fluid flow: The terms "fluid flow" and "bulk fluid flow" as used herein in connection with DLD refer to the macroscopic movement of fluid in a general direction across an obstacle array. These terms do not take into account the temporary displacements of fluid streams for fluid to move around an obstacle in order for the fluid to continue to move in the general direction.

Tilt angle ε: In a bump array device, the tilt angle is the angle between the direction of bulk fluid flow and the direction defined by alignment of rows of sequential (in the direction of bulk fluid flow) obstacles in the array.

Array Direction: In a bump array device, the "array direction" is a direction defined by the alignment of rows of sequential obstacles in the array. A particle is "bumped" in a bump array if, upon passing through a gap and encountering a downstream obstacle, the particle's overall trajectory follows the array direction of the bump array *(i.e.,* travels at the tilt angle ε relative to bulk fluid flow). A particle is not bumped if its overall trajectory follows the direction of bulk fluid flow under those circumstances.

### Detailed Description of the Invention

The present invention is concerned with the use of DLD in preparing cells that are of therapeutic value. The text below provides general guidance regarding methods disclosed herein and information that may aid in the making and use of devices involved in carrying out those methods.

### I. Designing Microfluidic Plates

Cells, particularly cells in compositions prepared by apheresis or leukapheresis, and particles may be isolated by performing DLD using microfluidic devices that contain a channel through which fluid flows from one or more inlets at or near one end of the device to outlets at or near the opposite end. Basic principles of size based microfluidic separations and the design of obstacle arrays for separating cells have been provided elsewhere (see, US 2014/0342375; US 2016/0139012; 7,318,902 and US 7,150,812) and are also summarized in the sections below.

During DLD, a fluid sample containing particles or cells is introduced into a device at an inlet and is carried along with fluid flowing through the device to outlets. As cells in the sample traverse the device, they encounter posts or other obstacles that have been positioned in rows and that form gaps or pores through which the cells must pass. Each successive row of obstacles is displaced relative to the preceding row so as to form an array direction that differs from the direction of fluid flow in the flow channel. The "tilt angle" defined by these two directions, together with the width of gaps between obstacles, the shape of obstacles, and the orientation of obstacles forming gaps are primary factors in determining a "critical size" for an array. Cells having a size greater than the critical size travel in the array direction, rather than in the direction of bulk fluid flow and particles having a size less than the critical size travel in the direction of bulk fluid flow. In devices used for apheresis or leukapheresis-derived compositions, array characteristics may be chosen that result in white blood cells being diverted in the array direction whereas red blood cells and platelets continue in the direction of bulk fluid flow. In order to separate a chosen type of leukocyte from others having a similar size, a carrier may then be used that binds to that cell in a way that promotes DLD separation and which thereby results in a complex that is larger than uncomplexed leukocytes. It may then be possible to carry out a separation on a device having a critical size smaller than the complexes but bigger than the uncomplexed cells.

The obstacles used in devices may take the shape of columns or be triangular, square, rectangular, diamond shaped, trapezoidal, hexagonal or teardrop shaped. In addition, adjacent obstacles may have a geometry such that the portions of the obstacles defining the gap are either symmetrical or asymmetrical about the axis of the gap that extends in the direction of bulk fluid flow.

### II. Making and Operating Microfluidic Devices

General procedures for making and using microfluidic devices that are capable of separating cells or particles on the basis of size are well known in the art. Such devices include those described in US 5,837,115; US 7,150,812; US 6,685,841; US 7,318,902; 7,472,794; and US 7,735,652. Other references that provide guidance that may be helpful in the making and use of devices for the present invention include: US 5,427,663; US 7,276,170; US 6,913,697; US 7,988,840; US 8,021,614; US 8,282,799; US8,304,230; US 8,579,117; US 2006/0134599; US 2007/0160503; US 20050282293; US 2006/0121624; US 2005/0266433; US 2007/0026381; US 2007/0026414; US 2007/0026417; US 2007/0026415; US 2007/0026413; US 2007/0099207; US 2007/0196820; US 2007/0059680; US 2007/0059718; US 2007/005916; US 2007/0059774; US 2007/0059781; US 2007/0059719; US 2006/0223178; US 2008/0124721; US 2008/0090239; US 2008/0113358; and WO2012094642. Of the various references describing the making and use of devices, US 7,150,812 provides particularly good guidance and 7,735,652 is of particular interest with respect to microfluidic devices for separations performed on samples with cells found in blood (in this regard, see also US 2007/0160503).

A device can be made using any of the materials from which micro- and nano-scale fluid handling devices are typically fabricated, including silicon, glasses, plastics, and hybrid materials. A diverse range of thermoplastic materials suitable for microfluidic fabrication is available, offering a wide selection of mechanical and chemical properties that can be leveraged and further tailored for specific applications.

Techniques for making devices include Replica molding, Soft lithography with PDMS, Thermoset polyester, Embossing, Injection Molding, Laser Ablation and combinations thereof. Further details can be found in "Disposable microfluidic devices: fabrication, function and application" by Fiorini, et al. (BioTechniques 38:429-446 (March 2005)). The book "Lab on a Chip Technology" edited by Keith E. Herold and Avraham Rasooly, Caister Academic Press Norfolk UK (2009) is another resource for methods of fabrication.

High-throughput embossing methods such as reel-to-reel processing of thermoplastics is an attractive method for industrial microfluidic chip production. The use of single chip hot embossing can be a cost-effective technique for realizing high-quality microfluidic devices during the prototyping stage. Methods for the replication of microscale features in two thermoplastics, polymethylmethacrylate (PMMA) and/or polycarbonate (PC), are described in "Microfluidic device fabrication by thermoplastic hot-embossing" by Yang, et al. (Methods Mol. Biol. 949: 115-23 (2013)).

The flow channel can be constructed using two or more pieces which, when assembled, form a closed cavity (preferably one having orifices for adding or withdrawing fluids) having the obstacles disposed within it. The obstacles can be fabricated on one or more pieces that are assembled to form the flow channel, or they can be fabricated in the form of an insert that is sandwiched between two or more pieces that define the boundaries of the flow channel.

The obstacles may be solid bodies that extend across the flow channel, in some cases from one face of the flow channel to an opposite face of the flow channel. Where an obstacle is integral with (or an extension of) one of the faces of the flow channel at one end of the obstacle, the other end of the obstacle can be sealed to or pressed against the opposite face of the flow channel. A small space (preferably too small to accommodate any particles of interest for an intended use) is tolerable between one end of an obstacle and a face of the flow channel, provided the space does not adversely affect the structural stability of the obstacle or the relevant flow properties of the device.

The number of obstacles present should be sufficient to realize the particle-separating properties of the arrays. The obstacles can generally be organized into rows and columns (Note: Use of the term "rows and columns" does not mean or imply that the rows and columns are perpendicular to one another). Obstacles that are generally aligned in a direction transverse to fluid flow in the flow channel can be referred to as obstacles in a column. Obstacles adjacent to one another in a column may define a gap through which fluid flows.

Obstacles in adjacent columns can be offset from one another by a degree characterized by a tilt angle, designated ε (epsilon). Thus, for several columns adjacent to one another *(i.e.,* several columns of obstacles that are passed consecutively by fluid flow in a single direction generally transverse to the columns), corresponding obstacles in the columns can be offset from one another such that the corresponding obstacles form a row of obstacles that extends at the angle ε relative to the direction of fluid flow past the columns. The tilt angle can be selected and the columns can be spaced apart from each other such that 1/ε (when expressed in radians) is an integer, and the columns of obstacles repeat periodically. The obstacles in a single column can also be offset from one another by the same or a different tilt angle. By way of example, the rows and columns can be arranged at an angle of 90 degrees with respect to one another, with both the rows and the columns tilted, relative to the direction of bulk fluid flow through the flow channel, at the same angle of ε.

Surfaces can be coated to modify their properties and polymeric materials employed to fabricate devices, can be modified in many ways. In some cases, functional groups such as amines or carboxylic acids that are either in the native polymer or added by means of wet chemistry or plasma treatment are used to crosslink proteins or other molecules. DNA can be attached to COC and PMMA substrates using surface amine groups. Surfactants such as Pluronic^{®} can be used to make surfaces hydrophilic and protein repellant by adding Pluronic^{®} to PDMS formulations. In some cases, a layer of PMMA is spin coated on a device, e.g., microfluidic chip and PMMA is "doped" with hydroxypropyl cellulose to vary its contact angle.

To reduce non-specific adsorption of cells or compounds, e.g., released by lysed cells or found in biological samples, onto the channel walls, one or more walls may be chemically modified to be non-adherent or repulsive. The walls may be coated with a thin film coating (e.g., a monolayer) of commercial non-stick reagents, such as those used to form hydrogels. Additional examples of chemical species that may be used to modify the channel walls include oligoethylene glycols, fluorinated polymers, organosilanes, thiols, poly-ethylene glycol, hyaluronic acid, bovine serum albumin, poly-vinyl alcohol, mucin, poly-HEMA, methacrylated PEG, and agarose. Charged polymers such as heparin may also be employed to repel oppositely charged species. The type of chemical species used for repulsion and the method of attachment to the channel walls can depend on the nature of the species being repelled and the nature of the walls and the species being attached. Such surface modification techniques are well known in the art. The walls may be functionalized before or after the device is assembled.

### III. Separation Processes that Use DLD

The DLD devices described herein can be used to purify cells, cellular fragments, cell adducts, or nucleic acids. As discussed herein, these devices can also be used to separate a cell population of interest from a plurality of other cells. Separation and purification of blood components using devices can be found, for example, in US Publication No. US2016/0139012. A brief discussion of a few illustrative separations is provided below.

### A. Viable Cells

In one embodiment devices are used in procedures designed to separate a viable cell from a nonviable cell. The term "viable cell" refers to a cell that is capable of growth, is actively dividing, is capable of reproduction, or the like. In instances where a viable cell has a size that is greater than a nonviable cell, DLD devices can be designed to comprise a critical size that is greater than a predetermined size of the nonviable cell and less than a predetermined size of the viable cell. The critical size may be as little as 1.1 fold greater than (or less than) the predetermined size of the nonviable cell but generally, larger degrees (or smaller) are preferred, e.g., about 1.2 fold - 2 fold, and preferably 3-10 fold.

### B. Adherent Cells

In another embodiment, DLD devices can be used to separate adherent cells. The term "adherent cell" as used herein refers to a cell capable of adhering to a surface. Adherent cells include immortalized cells used in cell culturing and can be derived from mammalian hosts. In some instances, the adherent cell may be trypsinized prior to purification. Examples of adherent cells include MRC-5 cells; HeLa cells; Vero cells; NIH 3T3 cells; L929 cells; Sf21 cells; Sf9 cells; A549 cells; A9 cells; AtT-20 cells; BALB/3T3 cells; BHK-21 cells; BHL-100 cells; BT cells; Caco-2 cells; Chang cells; Clone 9 cells; Clone M-3 cells; COS-1 cells; COS-3 cells; COS-7 cells; CRFK cells; CV-1 cells; D-17 cells; Daudi cells; GH1 cells; GH3 cells; HaK cells; HCT-15 cells; HL-60 cells; HT-1080 cells; HT-29 cells; HUVEC cells; I-10 cells; IM-9 cells; JEG-2 cells; Jensen cells; Jurkat cells; K-562 cells; KB cells; KG-1 cells; L2 cells; LLC-WRC 256 cells; McCoy cells; MCF7 cells; WI-38 cells; WISH cells; XC cells; Y-1 cells; CHO cells; Raw 264.7; BHK-21 cells; HEK 293 cells to include 293A, 293T and the like; HEP G2 cells; BAE-1 cells; SH-SY5Y cells; and any derivative thereof to include engineered and recombinant strains.

In some embodiments, procedures may involve separating cells from a diluent such as growth media, which may provide for the efficient maintenance of a culture of the adherent cells. For example, a culture of adherent cells in a growth medium can be exchanged into a transfection media comprising transfection reagents, into a second growth medium designed to elicit change within the adherent cell such as differentiation of a stem cell, or into sequential wash buffers designed to remove compounds from the culture.

In a preferred procedure, adherent cells are purified through association with one or more carriers that bind in a way that promotes DLD separation. The carriers may be of the type described herein and binding may stabilize and/or activate the cells. A carrier will typically be in the rage of 1-1000 µm but may sometimes also be outside of this range.

The association between a carrier and a cell should produce a complex of increased size relative to other material not associated with the carrier. Depending of the particular size of the cells and carriers and the number of cells and carriers present, a complex may be anywhere from a few percent larger than the uncomplexed cell to many times the size of the uncomplexed cell. In order to facilitate separations, an increase of at least 20% is desirable with higher percentages (50; 100; 1000 or more) being preferred.

### C. Activated Cells

The **DLD** devices can also be used in procedures for separating an activated cell or a cell capable of activation, from a plurality of other cells. The cells undergoing activation may be grown on a large scale but, in a preferred embodiment, the cells are derived from a single patient and DLD is performed within at least few hours after collection. The terms "activated cell" or "cell capable of activation" refers to a cell that has been, or can be activated, respectively, through association, incubation, or contact with a cell activator. Examples of cells capable of activation can include cells that play a role in the immune or inflammatory response such as: T cells, B cells; regulatory T cells, macrophages, dendritic cells, granulocytes, innate lymphoid cells, megakaryocytes, natural killer cells, thrombocytes, synoviocytes, and the like; cells that play a role in metabolism, such as beta cells, liver cells, and pancreatic cells; and recombinant cells capable of inducible protein expression such as DE3 lysogenized *E. coli* cells, yeast cells, plant cells, etc.

Typically, one or more carriers will have the activator on their surface. Examples of cell activators include proteins, antibodies, cytokines, CD3, CD28, antigens against a specific protein, helper T cells, receptors, and glycoproteins; hormones such as insulin, glucagon and the like; IPTG, lactose, allolactose, lipids, glycosides, terpenes, steroids, and alkaloids. The activatable cell should be at least partially associated with carriers through interaction between the activatable cell and cell activator on the surface of the carriers. The complexes formed may be just few percent larger than the uncomplexed cell or many times the size of the uncomplexed cell. In order to facilitate separations, an increase of at least 20% is desirable with higher percentages (40, 50 100 1000 or more) being preferred.

### D. Separating Cells from Toxic Material

DLD can also be used in purifications designed to remove compounds that may be toxic to a cell or to keep the cells free from contamination by a toxic compound. Examples include an antibiotic, a cryopreservative, an antifungal, a toxic metabolite, sodium azide, a metal ion, a metal ion chelator, an endotoxin, a plasticizer, a pesticide, and any combination thereof. The device can be used to remove toxic compounds from cells to ensure consistent production of material from the cells. In some instances, the cell can be a log phase cell. The term "log phase cell" refers to an actively dividing cell at a stage of growth characterized by exponential logarithmic growth. In log phase, a cell population can double at a constant rate such that plotting the natural logarithm of cell number against time produces a straight line.

The ability to separate toxic material may be important for a wide variety of cells including: bacterial strains such as BL21, Tuner, Origami, Origami B, Rosetta, C41, C43, DH5α, DH10β, or XL1Blue; yeast strains such as those of genera Saccharomyces, Pichia, Kluyveromyces, Hansenula and Yarrowia; algae; and mammalian cell cultures, including cultures of MRC-5 cells; HeLa cells; Vero cells; NIH 3T3 cells; L929 cells; Sf21 cells; Sf9 cells; A549 cells; A9 cells; AtT-20 cells; BALB/3T3 cells; BHK-21 cells; BHL-100 cells; BT cells; Caco-2 cells; Chang cells; Clone 9 cells; Clone M-3 cells; COS-1 cells; COS-3 cells; COS-7 cells; CRFK cells; CV-1 cells; D-17 cells; Daudi cells; GH1 cells; GH3 cells; HaK cells; HCT-15 cells; HL-60 cells; HT-1080 cells; HT-29 cells; HUVEC cells; I-10 cells; IM-9 cells; JEG-2 cells; Jensen cells; Jurkat cells; K-562 cells; KB cells; KG-1 cells; L2 cells; LLC-WRC 256 cells; McCoy cells; MCF7 cells; WI-38 cells; WISH cells; XC cells; Y-1 cells; CHO cells; Raw 264.7; BHK-21 cells; HEK 293 cells to include 293A, 293T and the like; HEP G2 cells; BAE-1 cells; SH-SY5Y cells; stem cells and any derivative thereof to include engineered and recombinant strains.

### V. Technological Background

Without being held to any particular theory, a general discussion of some technical aspects of microfluidics may help in understanding factors that affect separations carried out in this field. A variety of microfabricated sieving matrices have been disclosed for separating particles (Chou, et. al., Proc. Natl. Acad. Sci. 96:13762 (1999); Han, et al., Science 288:1026 (2000); Huang, et al., Nat. Biotechnol. 20:1048 (2002); Turner et al., Phys. Rev. Lett. 88(12):128103 (2002); Huang, et al., Phys. Rev. Lett. 89:178301 (2002); U.S. Pat. No. 5,427,663; U.S. Pat. No. 7,150,812; U.S. Pat. No. 6,881,317). Bump array (also known as "obstacle array") devices have been described, and their basic operation is explained, for example in U.S. Pat. No. 7,150,812. A bump array operates essentially by segregating particles passing through an array (generally, a periodically-ordered array) of obstacles, with segregation occurring between particles that follow an "array direction" that is offset from the direction of bulk fluid flow or from the direction of an applied field (U.S. 7,150,812).

### A. Bump Arrays

In some arrays, the geometry of adjacent obstacles is such that the portions of the obstacles defining the gap are symmetrical about the axis of the gap that extends in the direction of bulk fluid flow. The velocity or volumetric profile of fluid flow through such gaps is approximately parabolic across the gap, with fluid velocity and flux being zero at the surface of each obstacle defining the gap (assuming no-slip flow conditions) and reaching a maximum value at the center point of the gap. The profile being parabolic, a fluid layer of a given width adjacent to one of the obstacles defining the gap contains an equal proportion of fluid flux as a fluid layer of the same width adjacent to the other obstacle that defines the gap, meaning that the critical size of particles that are 'bumped' during passage through the gap is equal regardless of which obstacle the particle travels near.

In some cases, particle size-segregating performance of an obstacle array can be improved by shaping and disposing the obstacles such that the portions of adjacent obstacles that deflect fluid flow into a gap between obstacles are not symmetrical about the axis of the gap that extends in the direction of bulk fluid flow. Such lack of flow symmetry into the gap can lead to a non-symmetrical fluid flow profile within the gap. Concentration of fluid flow toward one side of a gap *(i.e.,* a consequence of the non-symmetrical fluid flow profile through the gap) can reduce the critical size of particles that are induced to travel in the array direction, rather than in the direction of bulk fluid flow. This is because the non-symmetry of the flow profile causes differences between the width of the flow layer adjacent to one obstacle that contains a selected proportion of fluid flux through the gap and the width of the flow layer that contains the same proportion of fluid flux and that is adjacent the other obstacle that defines the gap. The different widths of the fluid layers adjacent to obstacles define a gap that exhibits two different critical particle sizes. A particle traversing the gap can be bumped *(i.e.,* travel in the array direction, rather than the bulk fluid flow direction) if it exceeds the critical size of the fluid layer in which it is carried. Thus, it is possible for a particle traversing a gap having a non-symmetrical flow profile to be bumped if the particle travels in the fluid layer adjacent to one obstacle, but to be not-bumped if it travels in the fluid layer adjacent to the other obstacle defining the gap.

In another aspect, decreasing the roundness of edges of obstacles that define gaps can improve the particle size-segregating performance of an obstacle array. By way of example, arrays of obstacles having a triangular cross-section with sharp vertices can exhibit a lower critical particle size than do arrays of identically-sized and -spaced triangular obstacles having rounded vertices.

Thus, by sharpening the edges of obstacles defining gaps in an obstacle array, the critical size of particles deflected in the array direction under the influence of bulk fluid flow can be decreased without necessarily reducing the size of the obstacles. Conversely, obstacles having sharper edges can be spaced farther apart than, but still yield particle segregation properties equivalent to, identically-sized obstacles having less sharp edges.

### B. Fractionation Range

Objects separated by size on microfluidic devices include cells, biomolecules, inorganic beads, and other objects. Typical sizes fractionated range from 100 nanometers to 50 micrometers. However, larger and smaller particles may also sometimes be fractionated.

### C. Volumes

Depending on design, a device or combination of devices might be used to process between about 10 µl to at least 500 µl of sample, between about 500 µl and about 40 mL of sample, between about 500 µl and about 20 mL of sample, between about 20 mL of sample and about 200 mL of sample, between about 40 mL of sample and about 200 mL of sample, or at least 200 mL of sample. Total volume of material processed can, in some instances be between 50 ml and 5000 ml. 100 ml and 4000 ml or 500 and 2000 ml. Starting materials include blood, preparations derived from blood (e.g., apheresis or leukapheresis preparations), other biological fluids or extracts, cells grown in culture, etc.

### D. Channels

A device can comprise one or multiple channels with one or more inlets and one or more outlets. Inlets may be used for sample or crude *(i.e.,* unpurified) fluid compositions, for buffers or to introduce reagents. Outlets may be used for collecting product or may be used as an outlet for waste. Channels may be about 0.5 to 100 mm in width and about 2-200 mm long but different widths and lengths are also possible. Depth may be 1- 1000 µm and there may be anywhere from 1 to 100 channels or more present. Volumes may vary over a very wide range from a few µl to many ml and devices may have a plurality of zones (stages, or sections) with different configurations of obstacles.

### E. Gap Size (Edge-to-Edge Distance Between Posts or Obstacles)

Gap size in an array of obstacles (edge-to-edge distance between posts or obstacles) can vary from about a few *(e.g.,* 1-500) micrometers or be more than a millimeter. Obstacles may, in some embodiments have a diameter of 1-3000 micrometers and may have a variety of shapes (round, triangular, teardrop shaped, diamond shaped, square, rectangular etc.). A first row of posts can be located close to *(e.g.* within 5 µm) the inlet or be more than 1 mm away.

### F. Stackable chips

A device can include a plurality of stackable chips. A device can comprise about 1 - 50 chips. In some instances, a device may have a plurality of chips placed in series or in parallel or both.

### Examples

The following example is intended to illustrate, but not limit the invention.

A normal blood sample was diluted to 0.2x and processed on the DLD in a normal separation mode (running sample against buffer). The initial product fraction collected had the expected concentration based on the input counts and the DLD device ratios of inlets and outlets (further 0.28x dilution). Mid-way through the run, the collected product was recirculated into the DLD device as the "wash stream." Various fractions were collected at different time points as the DLD product continued to be recirculated. The final volume and concentration of the DLD product was compared to the input volume and concentration of the sample in order to obtain a recovery value. The net recovery of WBCs after multiple passes was 97.5%, and the concentration factor change from input material to product material was 2.9x (versus the initial change of 0.28x from input to product when processing the sample against running buffer).

Having now fully described the invention, it will be understood by one of skill in the art that the invention may be performed within a wide range of conditions, parameters and the like, without affecting the spirit or scope of the invention or any embodiment thereof.

## Claims

1. A method of separating target cells or target particles of a predetermined size from a sample comprising cells or particles of less than the predetermined size, the method comprising:
a) applying both the sample and a wash fluid to a microfluidic device at separate inlets, wherein:
i) the wash fluid applied to the device is devoid of said target cells or target particles and devoid of said cells or particles of less than the predetermined size;
ii) the microfluidic device comprises an array of obstacles arranged in rows, with each subsequent row of obstacles shifted laterally with respect to a previous row, and wherein the obstacles are positioned so as to differentially deflect target cells or particles to a first outlet where they may be recovered as a target cell or target particle product, and to direct the cells or particles of less than the predetermined size to a second outlet where they may be collected or discarded as waste;
iii) the first outlet comprises, or is connected to, a valve that can be used to divert the target cell or target particle product to a conduit that recycles the product to an inlet on the microfluidic device;
b) performing deterministic lateral displacement (DLD) by flowing the sample and wash fluid through the device, wherein during the performance of said DLD, at least a portion of the target cell or target particle product is recirculated one or more times so as to replace, all, or at least a portion, of the wash fluid being applied to the device;
c) during, or at the end of, step b), collecting a final product comprising target cells or particles from the first outlet.

2. The method of claim 1, wherein after the target cell or target particle product has been recirculated, recirculation is stopped and wash fluid is again applied to the microfluidic device.

3. The method of claim 1 or claim 2, wherein:
(a) the wash fluid is water or an aqueous buffer, and/or i) comprises reagents that chemically react with cells, particles or other components in the wash fluid; or ii) comprises antibodies, carriers or activators that interact specifically with target cells or target particles; and/or
(b) the microfluidic device comprises an inlet that comprises or is connected to a valve that can be used to switch the feed entering the device through the inlet from a conduit feeding wash buffer to a conduit feeding cell or target particle product; and/or
(c) the target cell or target particle product being recirculated to said microfluidic device is reacted with, or bound to, a carrier. antibody, fluorescent tag, activator or compound prior to, during or after being reapplied to the microfluidic device; and/or
(d) cell or particle counts are made of the target cell or target particle product; and/or
(e) recirculation is continued until, relative to the concentration in the sample, cells or particles are concentrated by a factor of at least 3, 5 or 10; and/or
(f) recirculation together with microfluidic processing is the sole method used for concentrating cells or particles.

4. The method of any one of claims 1-3, wherein the sample comprises target cells or stem cells of a predetermined size and cells less than the predetermined size, optionally wherein:
(a) the target cells are leukocytes and cells less than the predetermined size are platelets or red blood cells; and/or
(b) the sample is blood or a composition that has been obtained by performing apheresis or leukapheresis on blood; and/or
(c) the leukocytes or stem cells being recirculated are bound to a carrier, antibody, or activator in a way that promotes or complements DLD separation.

5. The method of any one of claims 4(a)-(c), wherein the leukocytes are T cells.

6. The method of claim 5, wherein said method is being used in a process for producing CAR-T cells, optionally wherein:
(a) said process does not include a centrifugation step; and/or
(b) said method is used to concentrate cells sufficiently to allow for their therapeutic administration to a patient, optionally wherein the sample has been obtained from a patient and no more than four hours elapse from the time that the sample has been obtained until DLD is completed.

7. A method of making purified genetically engineered target cells, comprising:
a) performing the method of any one of claims 1 to 5, wherein said recirculation is continued or repeated until a desired product cell concentration, PC, is reached;
b) once PC is reached, directing the flow of target cells from the first outlet to a site where the target cells are transformed or transfected to form genetically engineered target cells;
c) flowing the genetically engineered target cells to a device where they are separated from reagents, virus or other materials used in transforming or transfecting the target cells to form purified genetically engineered target cells;
d) either collecting the purified genetically engineered target cells or flowing the purified genetically engineered target cells to another site where they are further processed before collection.

8. The method of claim 7, wherein all steps from the applying of cells to the microfluidic device in step a) to the collection of cells in step d) is performed as a single continuous process.

9. The method of either claim 7 or claim 8, wherein the target cells are:
(a) lymphocytes or stem cells; or
(b) T lymphocytes, optionally wherein the T lymphocytes are genetically engineered to produce the chimeric antigen receptors (CARs) on their surface.

10. The method of any one of claims 7-9, wherein, in step c):
(a) the device is a microfluidic device and the genetically engineered target cells are separated from reagents, virus or other materials used in transforming or transfecting the target cells by DLD; and/or
(b) during the separation of reagents, virus or other materials used in transforming or transfecting the target cells, the purified genetically engineered target cells are transferred into growth medium and either collected in step d) and expanded in cell culture or are flowed to a site where they are cultured and then collected.

11. The method of any one of claims 7-10, wherein the first outlet comprises, or is connected to, a valve that can be used to direct the target cells to a conduit that recycles the product to an inlet on the microfluidic device or, alternatively can direct the flow of target cells from the first outlet to a site where the target cells are transformed or transfected to form genetically engineered target cells, optionally wherein the valve is configured electronically respond to a concentration of target cells below PC by directing the target cells to the conduit that recycles the product to an inlet on the microfluidic device and to electronically respond to a concentration of target cells at or above PC by directing the target cells to the site where the target cells are transformed or transfected to form genetically engineered target cells.

12. The method of any one of claims 7-11, wherein during the entire time after the sample has been obtained until the collection of cells in step d), target cells are not: (a) centrifuged; and/or (b) frozen.

13. The method of any one of claims 7-12, wherein the sample has been obtained from a patient and no more than 10 hours elapse from the time that the sample has been obtained until step d) is completed, optionally wherein no more than 5 hours elapse from the time that the sample has been obtained until step d) is completed.

## Patentansprüche

1. Verfahren zum Abtrennen von Zielzellen oder Zielpartikeln einer vorbestimmten Größe aus einer Probe, die Zellen oder Partikel umfasst, die kleiner als die vorbestimmte Größe sind, wobei das Verfahren umfasst:
a) Aufbringen sowohl der Probe als auch einer Waschflüssigkeit auf eine mikrofluidische Vorrichtung an getrennten Einlässen, wobei:
i) die auf die Vorrichtung aufgebrachte Waschflüssigkeit frei von den besagten Zielzellen oder Zielpartikeln und frei von den besagten Zellen oder Partikeln mit einer Größe unterhalb der vorbestimmten Größe ist;
ii) die mikrofluidische Vorrichtung eine Anordnung von Hindernissen umfasst, die in Reihen angeordnet sind, wobei jede nachfolgende Reihe von Hindernissen seitlich gegenüber einer vorherigen Reihe verschoben ist, und wobei die Hindernisse so positioniert sind, differenziell Zielzellen oder -partikel zu einem ersten Auslass abzulenken, wo sie als Zielzell- oder Zielpartikelprodukt gewonnen werden können, und die Zellen oder Partikel, die kleiner als die vorbestimmte Größe sind, zu einem zweiten Auslass zu leiten, wo sie gesammelt oder als Abfall entsorgt werden können;
iii) der erste Auslass ein Ventil umfasst oder mit einem Ventil verbunden ist, das verwendet werden kann, um das Zielzell- oder Zielpartikelprodukt zu einer Leitung umzuleiten, die das Produkt zu einem Einlass an der mikrofluidischen Vorrichtung wiederverwertet;
b) Ausführen einer deterministischen seitlichen Verschiebung (Deterministic Lateral Displacement, DLD) durch fließen lassen der Probe und der Waschflüssigkeit durch die Vorrichtung, wobei während der Ausführung der besagten DLD mindestens ein Teil des Zielzell- oder Zielpartikelprodukts ein- oder mehrmals rezirkuliert wird, um die gesamte, oder mindestens einen Teil der auf die Vorrichtung aufgebrachten Waschflüssigkeit zu ersetzen;
c) während oder am Ende von Schritt b), Sammeln eines Endprodukts, das Zielzellen oder -partikel umfasst, aus dem ersten Auslass.

2. Verfahren nach Anspruch 1, wobei, nachdem das Zielzell- oder Zielpartikelprodukt rezirkuliert wurde, das Rezirkulieren gestoppt wird und erneut Waschflüssigkeit auf die mikrofluidische Vorrichtung aufgebracht wird.

3. Verfahren nach Anspruch 1 oder Anspruch 2, wobei,
(a) die Waschflüssigkeit Wasser oder ein wässriger Puffer ist und/oder i) Reagenzien umfasst, die chemisch mit Zellen, Partikeln oder anderen Komponenten in der Waschflüssigkeit reagieren; oder ii) Antikörper, Träger oder Aktivatoren umfasst, die spezifisch mit Zielzellen oder Zielpartikeln interagieren; und/oder
(b) die mikrofluidische Vorrichtung einen Einlass umfasst, der ein Ventil umfasst oder mit einem Ventil verbunden ist, das verwendet werden kann, um die Zufuhr, die durch den Einlass in die Vorrichtung gelangt, von einer Leitung, die Waschpuffer zuführt, auf eine Leitung umzuschalten, die Zellen oder Zielpartikelprodukt zuführt; und/oder
(c) das Zielzell- oder Zielpartikelprodukt, das zu der mikrofluidischen Vorrichtung rezirkuliert wird, vor, während oder nach dem Wiederaufbringen auf die mikrofluidische Vorrichtung mit einem Träger, Antikörper, Fluoreszenzmarker, Aktivator oder einer Verbindung umgesetzt oder an diesen gebunden wird; und/oder.
(d) Zell- oder Partikelzählung des Zielzell- oder Zielpartikelprodukts durchgeführt wird; und/oder
(e) die Rezirkulation fortgesetzt wird, bis die Zellen oder Partikel im Verhältnis zur Konzentration in der Probe um einen Faktor von mindestens 3, 5 oder 10 konzentriert sind; und/oder
(f) die Rezirkulation zusammen mit der mikrofluidischen Verarbeitung das einzige Verfahren ist, das zur Konzentration von Zellen oder Partikeln verwendet wird.

4. Verfahren nach einem der Ansprüche 1 bis 3, wobei die Probe Zielzellen oder Stammzellen einer vorbestimmten Größe und Zellen kleiner als die vorbestimmte Größe umfasst, wobei optional:
(a) die Zielzellen Leukozyten sind und Zellen, die kleiner als die vorbestimmte Größe sind, Thrombozyten oder rote Blutkörperchen sind; und/oder
(b) die Probe Blut oder eine Zusammensetzung ist, die durch Ausführen von Apherese oder Leukapherese an Blut gewonnen wurde; und/oder
(c) die rezirkulierten Leukozyten oder Stammzellen an einen Träger, Antikörper oder Aktivator derart gebunden sind, dass dies die DLD-Trennung fördert oder ergänzt.

5. Verfahren nach einem der Ansprüche 4(a)-(c), wobei die Leukozyten T-Zellen sind.

6. Verfahren nach Anspruch 5, wobei das Verfahren in einem Prozess zur Herstellung von CAR-T-Zellen verwendet wird, wobei optional:
(a) der Prozess keinen Zentrifugationsschritt umfasst; und/oder
(b) das Verfahren verwendet wird, um Zellen ausreichend zu konzentrieren, um eine therapeutische Verabreichung an einen Patienten zu ermöglichen, wobei die Probe optional einem Patienten entnommen wurde, und nicht mehr als vier Stunden vergehen vom Zeitpunkt, zu dem die Probe entnommen wurde, bis die DLD abgeschlossen ist.

7. Verfahren zur Herstellung gereinigter gentechnisch veränderter Zielzellen, umfassend:
a) Ausführen des Verfahrens nach einem der Ansprüche 1 bis 5, wobei die Rezirkulation fortgesetzt oder wiederholt wird, bis eine gewünschte Produktzellkonzentration PC erreicht ist;
b) sobald PC erreicht ist, Leiten des Flusses der Zielzellen vom ersten Auslass zu einer Stelle, an der die Zielzellen transformiert oder transfiziert werden, um gentechnisch veränderte Zielzellen zu bilden;
c) Fließen lassen der gentechnisch veränderten Zielzellen zu einer Vorrichtung, wo sie von Reagenzien, Viren oder anderen Materialien abgetrennt werden, die bei der Transformation oder Transfektion der Zielzellen verwendet wurden, um gereinigte gentechnisch veränderte Zielzellen zu bilden;
d) entweder Sammeln der gereinigten gentechnisch veränderten Zielzellen oder Fließen lassen der gereinigten gentechnisch veränderten Zielzellen zu einer anderen Stelle, wo sie vor dem Sammeln weiterverarbeitet werden.

8. Das Verfahren nach Anspruch 7, wobei alle Schritte vom Aufbringen der Zellen auf die mikrofluidische Vorrichtung in Schritt a) bis zum Sammeln der Zellen in Schritt d) als ein einziger kontinuierlicher Prozess durchgeführt werden.

9. Verfahren nach entweder Anspruch 7 oder Anspruch 8, wobei die Zielzellen:
(a) Lymphozyten oder Stammzellen; oder
(b) T-Lymphozyten sind, wobei optional die T-Lymphozyten gentechnisch so verändert sind, dass sie auf ihrer Oberfläche die chimären Antigenrezeptoren (chimeric antigen receptors CARs) produzieren.

10. Verfahren nach einem der Ansprüche 7 bis 9, wobei in Schritt c):
(a) die Vorrichtung eine mikrofluidische Vorrichtung ist und die gentechnisch veränderten Zielzellen von Reagenzien, Viren oder anderen Materialien abgetrennt werden, die bei der Transformation oder Transfektion der Zielzellen durch DLD verwendet wurden; und/oder
(b) während der Abtrennung von Reagenzien, Viren oder anderen Materialien, die bei der Transformation oder Transfektion der Zielzellen verwendet wurden, die gereinigten gentechnisch veränderten Zielzellen in ein Wachstumsmedium überführt und entweder in Schritt d) gesammelt und in einer Zellkultur vermehrt werden oder zum Fließen zu einer Stelle gebracht werden, wo sie kultiviert und anschließend gesammelt werden.

11. Verfahren nach einem der Ansprüche 7 bis 10, wobei der erste Auslass ein Ventil umfasst oder mit einem Ventil verbunden ist, das verwendet werden kann, um die Zielzellen zu einer Leitung zu leiten, die das Produkt zu einem Einlass an der mikrofluidischen Vorrichtung wiederverwertet, oder alternativ den Fluss der Zielzellen vom ersten Auslass zu einer Stelle leiten kann, wo die Zielzellen transformiert oder transfiziert werden, um gentechnisch veränderte Zielzellen zu bilden, wobei optional das Ventil konfiguriert ist, elektronisch auf eine Konzentration von Zielzellen unterhalb von PC reagieren, indem es die Zielzellen zu der Leitung leitet, die das Produkt zu einem Einlass an der mikrofluidischen Vorrichtung wiederverwertet, und um elektronisch auf eine Konzentration von Zielzellen bei oder oberhalb von PC zu reagieren, indem es die Zielzellen zu der Stelle leitet, wo die Zielzellen transformiert oder transfiziert werden, um gentechnisch veränderte Zielzellen zu bilden.

12. Verfahren nach einem der Ansprüche 7 bis 11, wobei während der gesamten Zeit, nachdem die Probe entnommen wurde bis zum Sammeln der Zellen in Schritt d) die Zielzellen nicht: (a) zentrifugiert und/oder (b) eingefroren werden.

13. Verfahren nach einem der Ansprüche 7 bis 12, wobei die Probe einem Patienten entnommen wurde, und nicht mehr als 10 Stunden vergehen vom Zeitpunkt, zu dem die Probe entnommen wurde, bis Schritt d) abgeschlossen ist, wobei optional nicht mehr als 5 Stunden vergehen vom Zeitpunkt, zu dem die Probe entnommen wurde, bis Schritt d) abgeschlossen ist.

## Revendications

1. Procédé de séparation de cellules cibles ou de particules cibles d'une taille prédéterminée à partir d'un échantillon comprenant des cellules ou des particules d'une taille inférieure à la taille prédéterminée, le procédé comprenant le fait de :
a) appliquer à la fois l'échantillon et un fluide de lavage à un dispositif microfluidique au niveau d'entrées distinctes, dans lequel :
i) le fluide de lavage appliqué au dispositif est dépourvu desdites cellules cibles ou particules cibles et dépourvu desdites cellules ou particules dont la taille est inférieure à la taille prédéterminée ;
ii) le dispositif microfluidique comprend un réseau d'obstacles agencés en rangées, chaque rangée d'obstacles subséquente étant décalée latéralement par rapport à la rangée précédente, et dans lequel les obstacles sont positionnés de manière à dévier de façon différentielle les cellules cibles ou particules cibles vers une première sortie où elles peuvent être récupérées sous la forme d'un produit de cellules cibles ou de particules cibles, et à diriger les cellules ou particules dont la taille est inférieure à la taille prédéterminée vers une deuxième sortie où elles peuvent être collectées ou éliminées en tant que déchets ;
iii) la première sortie comprend, ou est reliée à, une vanne qui peut être utilisée pour détourner le produit de cellules cibles ou de particules cibles vers un conduit qui recycle le produit vers une entrée du dispositif microfluidique ;
b) effectuer un déplacement latéral déterministe (DLD) en faisant s'écouler l'échantillon et le fluide de lavage à travers le dispositif, dans lequel, pendant la réalisation dudit DLD, au moins une partie du produit de cellules cibles ou de particules cibles est recirculée une ou plusieurs fois de manière à remplacer la totalité, ou au moins une partie, du fluide de lavage appliqué au dispositif;
c) pendant, ou à la fin de, l'étape b), collecter un produit final comprenant des cellules cibles ou des particules cibles à partir de la première sortie.

2. Procédé selon la revendication 1, dans lequel, après la recirculation du produit de cellules cibles ou de particules cibles, la recirculation est arrêtée et le fluide de lavage est à nouveau appliqué au dispositif microfluidique.

3. Procédé selon la revendication 1 ou 2, dans lequel :
(a) le fluide de lavage est de l'eau ou un tampon aqueux, et/ou i) comprend des réactifs qui réagissent chimiquement avec les cellules, les particules ou d'autres composants du fluide de lavage ; ou ii) comprend des anticorps, des vecteurs ou des activateurs qui interagissent spécifiquement avec les cellules cibles ou les particules cibles ; et/ou
(b) le dispositif microfluidique comprend une entrée qui comprend, ou est reliée à, une vanne qui peut être utilisée pour commuter l'alimentation entrant dans le dispositif à travers l'entrée, d'un conduit alimentant un tampon de lavage à un conduit alimentant le produit de cellules cibles ou de particules cibles ; et/ou
(c) le produit de cellules cibles ou de particules cibles recirculé vers ledit dispositif microfluidique est mis en réaction avec, ou lié à, un vecteur, un anticorps, un marqueur fluorescent, un activateur ou un composé avant, pendant ou après qu'il a été réappliqué au dispositif microfluidique ; et/ou
(d) un comptage des cellules ou des particules est effectué sur le produit de cellules cibles ou de particules cibles ; et/ou
(e) la recirculation se poursuit jusqu'à ce que, par rapport à la concentration dans l'échantillon, les cellules ou les particules soient concentrées d'un facteur d'au moins 3, 5 ou 10 ; et/ou
(f) la recirculation conjointement avec un traitement microfluidique est le seul procédé utilisé pour concentrer les cellules ou les particules.

4. Procédé selon l'une quelconque des revendications 1 à 3, dans lequel l'échantillon comprend des cellules cibles ou des cellules souches d'une taille prédéterminée et des cellules dont la taille est inférieure à la taille prédéterminée, éventuellement dans lequel :
(a) les cellules cibles sont des leucocytes et les cellules dont la taille est inférieure à la taille prédéterminée sont des plaquettes ou des globules rouges ; et/ou
(b) l'échantillon est du sang ou une composition qui a été obtenue en effectuant une aphérèse ou une leucaphérèse sur du sang ; et/ou
(c) les leucocytes ou les cellules souches recirculé(e)s sont lié(e)s à un vecteur, un anticorps ou un activateur d'une manière qui favorise ou complète la séparation DLD.

5. Procédé selon l'une quelconque des revendications 4(a) à (c), dans lequel les leucocytes sont des cellules T.

6. Procédé selon la revendication 5, dans lequel ledit procédé est utilisé dans un processus de production de cellules CAR-T, éventuellement dans lequel :
(a) ledit processus ne comprend pas d'étape de centrifugation ; et/ou
(b) ledit procédé est utilisé pour concentrer les cellules suffisamment pour permettre leur administration thérapeutique à un patient, éventuellement dans lequel l'échantillon a été prélevé sur un patient et pas plus de quatre heures s'écoulent entre le moment où l'échantillon a été prélevé et l'achèvement du DLD.

7. Procédé de fabrication de cellules cibles génétiquement modifiées purifiées, comprenant le fait de :
a) mettre en œuvre le procédé selon l'une quelconque des revendications 1 à 5, dans laquelle ladite recirculation se poursuit ou se répète jusqu'à ce qu'une concentration souhaitée en cellules de produit, PC, soit atteinte ;
b) une fois la PC atteinte, diriger le flux de cellules cibles depuis la première sortie vers un site où les cellules cibles sont transformées ou transfectées pour former des cellules cibles génétiquement modifiées ;
c) faire s'écouler les cellules cibles génétiquement modifiées vers un dispositif où elles sont séparées des réactifs, des virus ou d'autres matériaux utilisés pour transformer ou transfecter les cellules cibles afin de former des cellules cibles génétiquement modifiées purifiées ;
d) soit collecter les cellules cibles génétiquement modifiées purifiées, soit faire s'écouler les cellules cibles génétiquement modifiées purifiées vers un autre site où elles sont traitées davantage avant d'être collectées.

8. Procédé selon la revendication 7, dans lequel toutes les étapes, depuis l'application des cellules au dispositif microfluidique à l'étape a) jusqu'à la collecte des cellules à l'étape d), sont effectuées en tant qu'un unique processus continu.

9. Procédé selon la revendication 7 ou 8, dans lequel les cellules cibles sont :
(a) des lymphocytes ou des cellules souches ; ou
(b) des lymphocytes **T,** dans lesquels, éventuellement, les lymphocytes T sont génétiquement modifiés pour produire des récepteurs antigéniques chimériques (CAR) à leur surface.

10. Procédé selon l'une quelconque des revendications 7 à 9, dans lequel, à l'étape c) :
(a) le dispositif est un dispositif microfluidique et les cellules cibles génétiquement modifiées sont séparées des réactifs, des virus ou d'autres matériaux utilisés pour transformer ou transfecter les cellules cibles par DLD ; et/ou
(b) pendant la séparation des réactifs, des virus ou d'autres matériaux utilisés pour transformer ou transfecter les cellules cibles, les cellules cibles génétiquement modifiées purifiées sont transférées dans un milieu de croissance, et sont soit collectées à l'étape d) et développées dans une culture cellulaire, soit transférées vers un site où elles sont cultivées puis collectées.

11. Procédé selon l'une quelconque des revendications 7 à 10, dans lequel la première sortie comprend, ou est reliée à, une vanne qui peut être utilisée pour diriger les cellules cibles vers un conduit qui recycle le produit vers une entrée du dispositif microfluidique ou, en variante, peut diriger le flux de cellules cibles depuis la première sortie vers un site où les cellules cibles sont transformées ou transfectées pour former des cellules cibles génétiquement modifiées, éventuellement, dans lequel la vanne est configurée pour répondre électroniquement à une concentration de cellules cibles inférieure à PC, en dirigeant les cellules cibles vers le conduit qui recycle le produit vers une entrée du dispositif microfluidique et pour répondre électroniquement à une concentration de cellules cibles égale ou supérieure à PC, en dirigeant les cellules cibles vers le site où les cellules cibles sont transformées ou transfectées pour former des cellules cibles génétiquement modifiées.

12. Procédé selon l'une quelconque des revendications 7 à 11, dans lequel, pendant toute la durée entre le prélèvement de l'échantillon et la collecte des cellules à l'étape d), les cellules cibles ne sont pas : (a) centrifugées ; et/ou (b) congelées.

13. Procédé selon l'une quelconque des revendications 7 à 12, dans lequel l'échantillon a été prélevé sur un patient et pas plus de 10 heures s'écoulent entre le moment où l'échantillon a été prélevé et l'achèvement de l'étape d), dans lequel, éventuellement, pas plus de 5 heures s'écoulent entre le moment où l'échantillon a été prélevé et l'achèvement de l'étape d).
